(19) Europäisches Patentamt
European Patent Office
Office européen des brevets

(11) **EP 3 325 571 B1**

(12) **EUROPEAN PATENT SPECIFICATION**

(45) Date of publication and mention of the grant of the patent:
**01.05.2019 Bulletin 2019/18**

(21) Application number: **16733304.6**

(22) Date of filing: **29.06.2016**

(51) Int Cl.:
*C09K 11/02* (2006.01)    *C09K 11/88* (2006.01)
*C09K 11/56* (2006.01)

(86) International application number:
**PCT/EP2016/001106**

(87) International publication number:
**WO 2017/012688 (26.01.2017 Gazette 2017/04)**

(54) **LUMINESCENT PARTICLE, INK FORMULATION, POLYMER COMPOSITION, OPTICAL DEVICE, FABRICATION THEREOF, AND USE OF THE LUMINESCENT PARTICLE**

LUMINESZIERENDES PARTIKEL, TINTENFORMULIERUNG, POLYMERZUSAMMENSETZUNG, OPTISCHE VORRICHTUNG, HERSTELLUNG DAVON UND VERWENDUNG DES LUMINESZIERENDEN PARTIKELS

PARTICULE LUMINESCENTE, FORMULATION D'ENCRE, COMPOSITION POLYMÈRE, DISPOSITIF OPTIQUE, FABRICATION DE CEUX-CI, ET UTILISATION DE LA PARTICULE LUMINESCENTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2015 EP 15177183**

(43) Date of publication of application:
**30.05.2018 Bulletin 2018/22**

(73) Proprietor: **Merck Patent GmbH**
**64293 Darmstadt (DE)**

(72) Inventors:
• **HIRAYAMA, Yuki**
**Machida**
**Tokyo 194-0215 (JP)**
• **OKURA, Hiroshi**
**Hiratsuka-shi**
**Kanagawa 254-0821 (JP)**
• **DERTINGER, Stephan**
**Tokyo 158-0091 (JP)**
• **KOBAYASHI, Yoshio**
**Hitachi**
**Ibaraki 316-0003 (JP)**

(56) References cited:
**CN-A- 103 911 142    US-A1- 2005 051 771**
**US-B1- 6 660 379**

• **SHIRAISHI Y ET AL: "Effects of poly-N-isopropylacrylamide on fluorescence properties of CdS/Cd(OH)2 nanoparticles in water", JOURNAL OF PHOTOCHEMISTRY AND PHOTOBIOLOGY, A: CHEMISTRY, ELSEVIER SEQUOIA, LAUSANNE, CH, vol. 205, no. 1, 10 June 2009 (2009-06-10) , pages 51-56, XP026150464, ISSN: 1010-6030, DOI: 10.1016/J.JPHOTOCHEM.2009.04.001 [retrieved on 2009-04-14]**

## Description

### Field of the Invention

The present invention relates to a luminescent particle, ink formulation, polymer composition, optical device and a preparation thereof. The invention further relates to use of a luminescent particle in an optical device or in biological imaging.

### Background Art

Luminescent particles comprising a nanosized phosphor, are used in a variety of optical applications or in biological imaging.

For example, as described in Francesca Pietra et al., Chem. Mater. 2013, 25, 3427 - 3434, JP 4568862 B, WO 2013/035362 A1, WO 2007/034877 A1, WO 2014/140936 A2, WO 2011/036447 A1, Yoshio Kobayashi et al.,J Sol-Gel Sci Technol (2010) 55:79-85, Yoshio Kobayashi et al.,J Sol-Gel Sci Technol (2013) 66:31-37, CN103911142 A.

### Patent Literature

1. JP 4568862 B
2. WO 2013/035362 A1
3. WO 2007/034877 A1
4. WO 2014/140936 A2
5. WO 2011/036447 A1

### Non patent Literature

6. Francesca Pietra et al., Chem. Mater. 2013, 25, 3427 - 3434
7. Yoshio Kobayashi et al.,J Sol-Gel Sci Technol (2010) 55:79-85
8. Yoshio Kobayashi et al.,J Sol-Gel Sci Technol (2013) 66:31-37

### Summary of the invention

However, the inventors newly have found that there is still one or more of considerable problems for which improvement is desired, as listed below.

1. A novel luminescent particle comprising a nanosized phosphor and a shell layer, which can show reduced and / or restrained quantum yield drop and better photoluminescence quantum yield is desired.

2. A luminescent particle comprising a novel structure to protect a nanosized phosphor from wet-fabrication process damage when fabricate a shell layer over the nanosized phosphor, is required.

3. Simple wet-fabrication process for a luminescent particle is also desired.

The inventors aimed to solve the problems mentioned above. Surprisingly, the inventors have found a novel luminescent particle (100), wherein the luminescent particle (100) comprises a nanosized phosphor (110), a polymer material (120) covering the nanosized phosphor (110), and a shell layer (130) covering the polymer material (120), wherein the shell layer (130) is selected from the group consisting of a metal hydroxide, metal carbonate hydroxide, metal polyphosphate and a combination of any of these, solves all of the problems 1 to 3 at the same time.

In another aspect, the invention relates to use of the luminescent particle (100), in an optical device or biological imaging.

In another aspect, the invention further relates to ink formulation comprising the luminescent particle (100), and solvent.

In another aspect, the invention further relates to a polymer composition comprising the luminescent particle (100), and a polymer matrix.

In another aspect, the invention further relates to an optical device comprising the luminescent particle (100).
In another aspect, the present invention furthermore relates to a method for preparing of the luminescent particle (100), wherein the method comprises the following sequential steps (a) and (b);

(a) mixing the nanosized phosphor and the polymer in solvent

(b) mixing the polymer covered nanosized phosphor obtained from step (a) and a precursor of the shell layer in solvent.

In another aspect, the present invention furthermore relates to a method for preparing the ink formulation, wherein the method comprises the step (X):
(X) mixing the luminescent particle and a solvent.
In another aspect, the present invention furthermore relates to method for preparing the polymer composition, wherein the method comprises the step (A):

(A) mixing the luminescent particle and a polymer.

Further advantages of the present invention will become evident from the following detailed description.

Description of drawings

Fig. 1: shows a cross sectional view of a schematic of one embodiment of a luminescent particle of the present invention.

Fig. 2: shows a cross sectional view of a schematic of one embodiment of an optical device of the invention.

Fig. 3: shows a cross sectional view of a schematic of another embodiment of an optical device of the invention.

Fig. 4: shows a cross sectional view of a schematic of another embodiment of an optical device of the invention.

Fig. 5: shows a cross sectional view of a schematic of another embodiment of an optical device of the invention.

Fig. 6: shows a cross sectional view of a schematic of another embodiment of an optical device of the invention.

Fig. 7: shows a cross sectional view of a schematic of another embodiment of an optical device of the invention.

Fig. 8: shows a cross sectional view of a schematic of another embodiment of an optical device of the invention.

Fig. 9: shows a TEM image of sample 1 in working example 3.

Fig. 10: shows a TEM image of sample 2 in working example 3.

Fig. 11: shows a TEM image of sample 3 in working example 3.

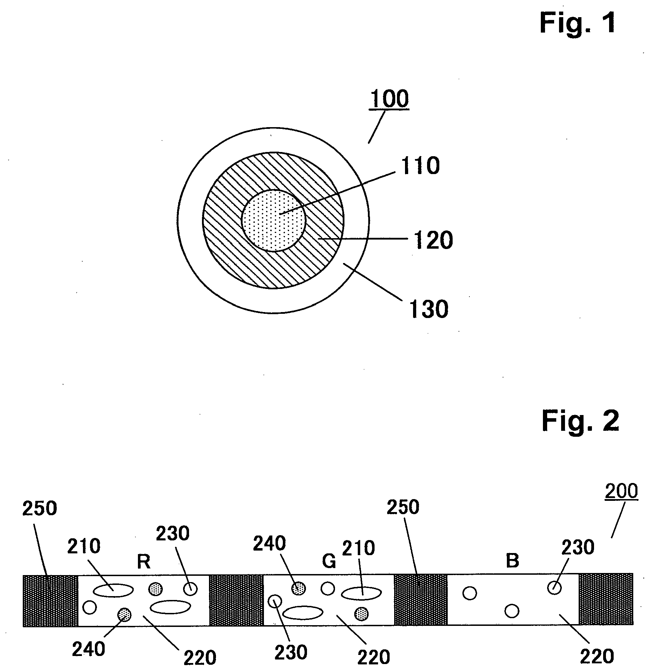

List of reference signs in figure 1

100. a luminescent particle
110. a nanosized phosphor
120. a polymer material
130. a shell layer

List of reference signs in figure 2

200. a color conversion film
210. a luminescent particle
220. a polymer matrix
230. a light scattering particle (optional)
240. a coloring agent (optional)
250. a black matrix (optional)

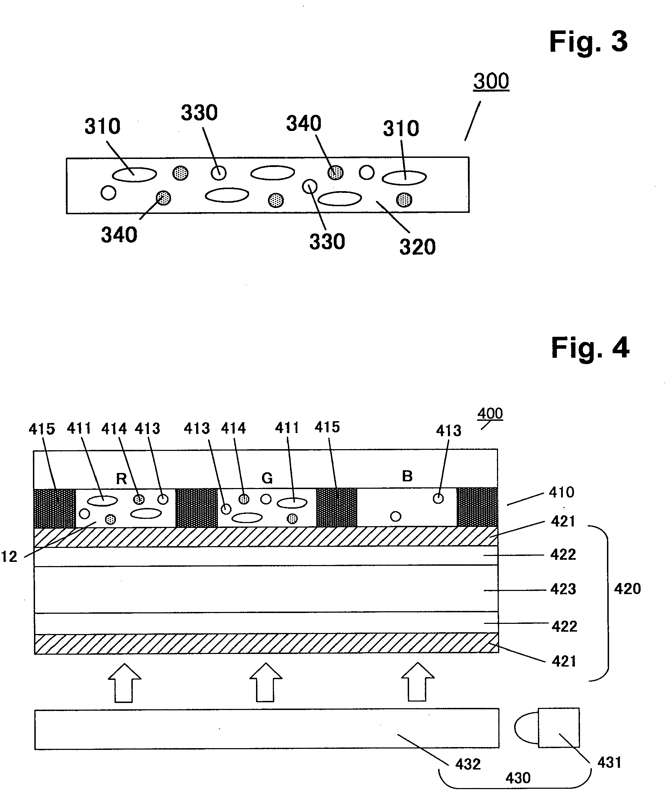

List of reference signs in figure 3

300. a color conversion film
310. a luminescent particle
320. a polymer matrix
330. a light scattering particle (optional)
340. a coloring agent (optional)

List of reference signs in figure 4

400. an optical device
410. a color conversion film
411. a luminescent particle
412. a polymer matrix
413. a light scattering particle (optional)
414. a coloring agent (optional)
415. a black matrix (optional)
420. a light modulator
421. a polarizer
422. an electrode
423. a liquid crystal layer
430. a light source
431. a LED light source
432. a light guiding plate (optional)

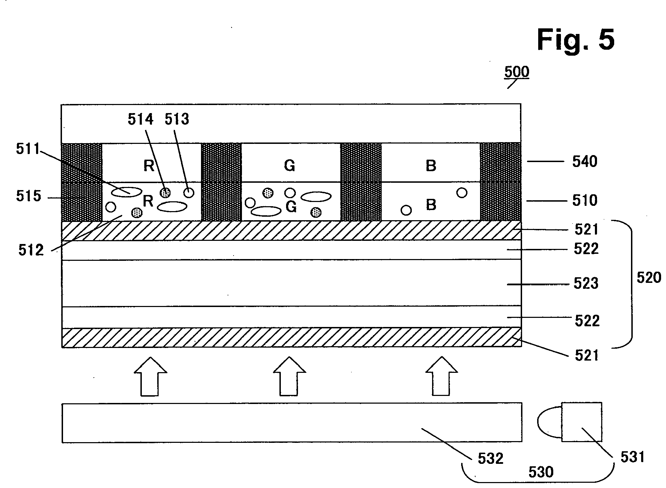

List of reference signs in figure 5

500. an optical device
510. a color conversion film
511. a luminescent particle
512. a polymer matrix
513. a light scattering particle (optional)
514. a coloring agent (optional)
515. a black matrix (optional)
520. a light modulator
521. a polarizer
522. an electrode
523. a liquid crystal layer
530. a light source
531. a LED light source
532. a light guiding plate (optional)
540. a color filter (optional)

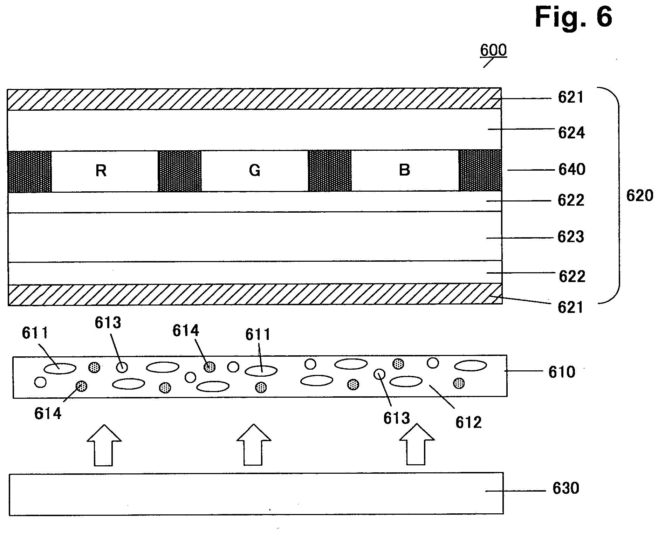

List of reference signs in figure 6

600. an optical device
610. a color conversion film
611. a luminescent particle
612. a polymer matrix
613. a light scattering particle (optional)
614. a coloring agent (optional)
620. a light modulator
621. a polarizer
622. an electrode
623. a liquid crystal layer
630. a light source
640. a color filter

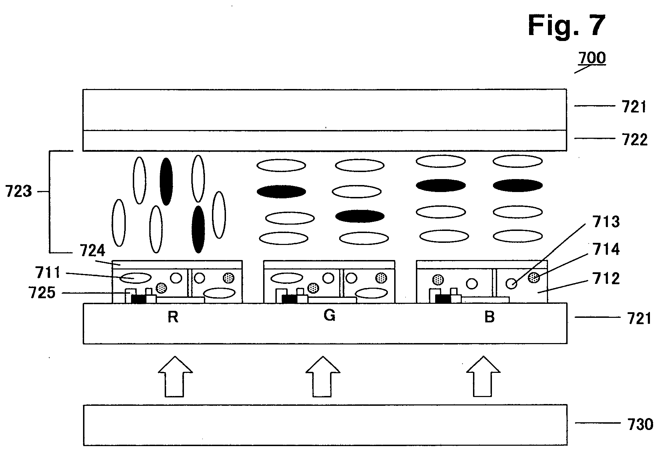

List of reference signs in figure 7

700. an optical device
710. a color conversion film fabricated into a light modulator
711. a luminescent particle
712. a polymer matrix
713. a light scattering particle (optional)
714. a coloring agent (optional)
720. a light modulator
721. a transparent substrate
722. a transparent electrode
723. a LC layer (doped with Dichroic Dye)
724. a transparent pixel electrode
725. a TFT (Thin film transistor)
730. a light source

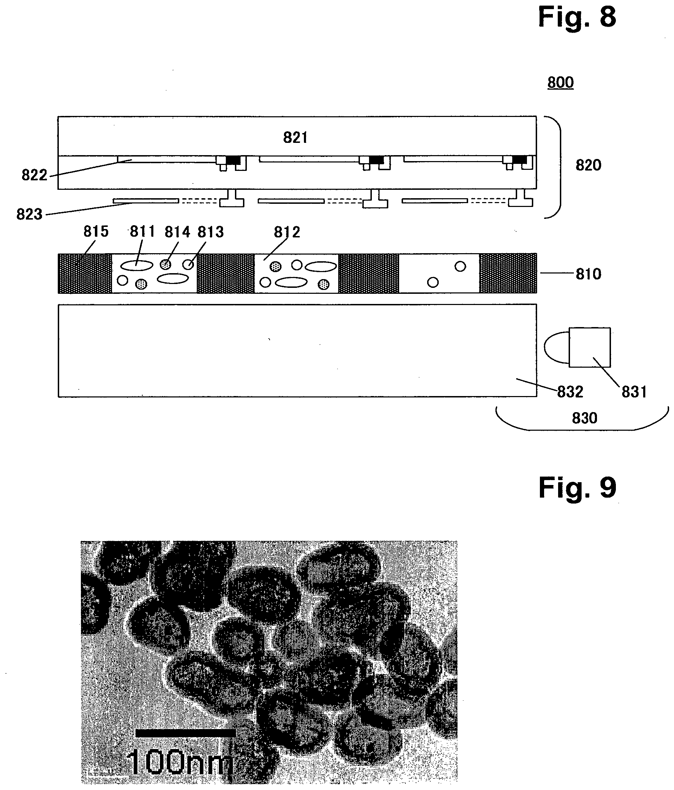

List of reference signs in figure 8

800. an optical device
810. a color conversion film
811. a luminescent particle
812. a polymer matrix
813. a light scattering particle (optional)
814. a coloring agent (optional)
815. a black matrix (optional)
820. a light modulator
821. a transparent substrate
822. a TFT (Thin film transistor)
823. MEMS (Micro Electro Mechanical Systems) Shutter
830. a light source
831. a LED light source
832. a light guiding plate (optional)

Detailed Description of the invention

In one aspect of the present invention, a luminescent particle (100), wherein the luminescent particle (100) comprises a nanosized phosphor (110), a polymer material (120) covering the nanosized phosphor (110), and a shell layer (130) covering the polymer material (120), wherein the shell layer (130) is selected from the group consisting of a metal hydroxide, metal carbonate hydroxide, metal polyphosphate and a combination of any of these, is provided by the inventors to solve whole problems 1 to 3 at the same time.

- Polymer material (120)

According to the present invention, a wide variety of publically known polymers can be used preferably as desired.

In some embodiments of the present invention, the polymer material (120) can be selected from the group consisting of a thermosetting resin, thermoplastics resin, and a combination of any of these.

Type of a thermosetting resin and / or a thermoplastics resin are not particularly limited. For example, as a thermoplastics resin, poly vinyl chloride, poly vinylidene chloride, poly vinyl acetate, poly vinyl alcohol, poly vinyl propionate, poly styrene, styrene-butadiene copolymer, styreneacrylonitrile copolymer, styrene-butadiene-acrylonitrile copolymer, ethylenevinyl acetate copolymer, poly ethylene, polyethylenimine, poly propylene, polyethyleneglycol, poly-isoprene, polyacetal, poly isoprene, poly butadiene, poly isobutene, poly butene, poly oxyethylene, poly oxypropylene, poly vinylethyl ether, polyvinylhexyl ether, poly vinyl butyl ether, poly acrylo nitrile, poly methacrylonitrile, poly sulfone, poly sulphide, phenoxy resins, polyethylacrylate, poly (meth)acrylates such as poly methyl methacrylate,

According to the present invention the term "polymer" means a material having a repeating unit and having the weight average molecular weight (Mw) 1000 or more.

In a preferred embodiment of the present invention, the weight average molecular weight (Mw) of the polymer (120) is in the range from 1,000 to 100,000. More preferably it is from 5,000 to 50,000. Even more preferably it is from 10,000 to 40,000.

Thus, in one embodiment of the present invention, the weight average molecular weight (Mw) of the polymer (120) is in the range from 1,000 to 100,000.

The weight average molecular weight (Mw) of the polymer (120) can be measured with Static Light Scattering Spectrophotometer "Zetasizer Nano ZS" (Malvern)

In a preferred embodiment of the present invention, the polymer (120) can further comprise an anchoring group selected from the group consisting of phosphate, phosphine, phosphine oxide, phosphonate, thiol, amino, carboxylate, carboxylic ester, hetero cycle, silane, sulfonate, hydroxyl and a combination of any of these, with more preferably being of amino, phosphate, carboxylate, or a combination of any of these.

Therefore, in some embodiments of the present invention, the polymer (120) further comprises an anchoring group selected from the group consisting of phosphate, phosphine, phosphine oxide, phosphonate, thiol, amino, carboxylate, carboxylic ester, hetero cycle, silane, sulfonate, hydroxyl and a combination of any of these.

And in one embodiment of the present invention, preferably, the anchoring group is amino, phosphate, carboxylate, or a combination of any of these.

In one embodiment of the present invention, preferably, the polymer (120) is a branched polymer.

Without wishing to be bound by theory, it is believed that the polymer (100) may lead to reduced and / or restrained quantum yield drop of the luminescent particle (100) and the branched polymer is more preferable to prevent or reduce wet-process fabrication damage of nanosized phosphor (110).

According to the present invention, the term "branched polymer" means a polymer having at least one branching point where a second chain of monomers branched off from the first chain.

In a preferred embodiment of the present invention, the branched polymer can be selected from the group consisting of a dendrimer, dendronized polymer, hyperbranched polymer, a polymer brush, a star polymer, and a combination of any of these.

For examples, as a polymer brush, DisperBYK-182, 184,190, 194; as a star polymer, Disperbyk-2090, 2091; as a dendrimer, PAMAM dendrimer product No. 412376, 635472, 635553, 526142, 412392, 412414, 412430, 470457, 536784, 536792, 597309, 597635, 595861, 596426, 596639, 596965, 647829, 648159, 412368, 412384, 536776, 412406, 412422, 412449, 536709, 436717, 536725, 536741, or 536768 (from Sigma-Aldrich); as a hyperbranched polymer, polyethylenimine (hereafter "PEI") can be used preferably.

According to the present invention, the term "dendronized polymer" means a polymer having a linear polymer chain in which dendrons are regularly branched onto the linear polymer chain.

According to the present invention, the term "Dendron" taken to mean that a polymer repetitively branched but not symmetrically branched around the core.

The term "Dendrimer" means a polymer having a core and symmetrically and repetitively branched around the core.

According to the present invention, the term "hyperbranched polymer" taken to mean that a polymer having one or more of 1st branching points on the first chains and at least one of second chains of monomers branched off from the first chains also has at least one or more of 2nd branching points, here the term "hyperbranched polymer" does not include "dendronized polymers" and "Dendrimers".

The hyperbranched polymers, according to the invention, can be characterized by a degree of branching (DB) which represents the percentage of dendritic and terminal monomers among the total monomers in the polymer and represented by the following formula (1);

$$DB = D + T / (D + T + L) * 100 \% \quad \text{- formula (1)}$$

(wherein the formula, the symbol "D" means the number of branching points in a polymer, the symbol "T" is the number of terminal parts in a polymer, and the symbol "L" is number of the unbranched parts in a polymer. Like described in Mitsuru Ueda, Landfall vol. 77, 2013, pp 16 - 21)

More preferably, the branched polymer can be a dendrimer, dendronized polymer, hyperbranched polymer or a combination of any of these.

Preferably, the polymer (120) is a branched polymer comprising an anchoring group selected from the group consisting of phosphate, phosphine, phosphine oxide, phosphonate, thiol, amino, carboxylate, carboxylic ester, hetero cycle, silane, sulfonate, hydroxyl and a combination of any of these, with more preferably being of amino, phosphate, carboxylate, or a combination of any of these.

In a preferred embodiment of the present invention, the polymer (120) can be a branched polymer selected from the group consisting of a dendrimer, dendronized polymer, hyperbranched polymer or a combination of any of these, wherein the polymer (120) comprising an anchoring group selected from the group consisting of phosphate, phosphine, phosphine oxide, phosphonate, thiol, amino, carboxylate, carboxylic ester, hetero cycle, silane, sulfonate, hydroxyl and a combination of any of these, with more preferably being of amino, phosphate, carboxylate, or a combination of any of these, and wherein the weight average molecular weight (Mw) of the polymer (120) is in the range from 1,000 to 100,000, with being more preferably in the range from 5,000 to 50,000. Even more preferably it is from 10,000 to 40,000.

-Shell layer (130)

According to the present invention, the shell layer is selected from the group consisting of a metal hydroxide, metal carbonate hydroxide, metal polyphosphate and a combination of any of these.

The types of metal hydroxide, metal carbonate hydroxide and metal polyphosphate are not particularly limited.

According to the present invention, the term "metal hydroxide" means a metal having at least one of hydroxide group. And the term "metal hydroxide" comprises a metal oxide-hydroxide, a hydrated metal oxide and a combination of metal oxide-hydroxide and a hydrated metal oxide.

For example, a silicone oxide hydroxide, aluminum oxide hydroxide, aluminum hydroxide, titanium oxide hydroxide, gadolinium oxide hydroxide, gadolinium hydroxide, copper oxide hydroxide, cupper hydroxide, zinc oxide hydroxide, zinc hydroxide, iron oxide hydroxide, gadolinium carbonate hydroxide, cupper carbonate hydroxide, zinc carbonate hydroxide and a combination of any of these.

In some embodiments of the present invention, metal hydroxide can comprise at least one or more of alkoxide groups.

In one embodiment of the present invention, the shell layer is amorphous or comprises fine crystallites.

According to the present inventioin, the term "fine crystallites" is taken to mean that it has micron sized and / or nanosized grains within a phase of the shell layer.

And the term "amorphous" means non-crystalline without long-range molecular order, the measured X-ray powder diffraction (XRPD) pattern of "amorphous" material is essentially continuous in appearance.

Without wishing to be bound by theory, it is believed that the shell layer (130) may lead to higher durability of the luminescent particle (100).

In some embodiments of the present invention, the shell layer can be two or more of stacked layers.

-Nanosized phosphor (110)

According to the present invention, any kind of publically known nanosized phosphor can be used preferably.

According to the present invention, the term "phosphor" means a material which can absorb excitation light and emits light having a longer peak wavelength than the peak wavelength of the excitation light.

According to the present invention, the term "nanosized" means the size in between 1 nm and 900 nm.

Thus, according to the present invention, the nanosized phosphor is taken to mean that the fluorescent material which size of the overall diameter is in the range from 1 nm to 900 nm. And in case of the material has elongated shape, the length of the overall structures of the fluorescent material is in the range from 1 nm to 900 nm.

In a preferred embodiment of the present invention, the nanosized phosphor can be selected from the group consisting of nanosized inorganic phosphor material, quantum sized material such as quantum dot and or quantum rod, and a combination of any of these.

Thus, in one embodiment of the present invention, the nanosized phosphor is a nanosized inorganic phosphor, or a quantum sized material.

For example, as a nanosized inorganic phosphor, (YAG:Ce) yellow nanophosphor, $Bi^{3+}$,$Eu^{3+}$ co-activated Yttrium Vanadate ($YVO_4$) red nanophosphor, ZnS:Mn and (Zn,Cd):Mn red nanophosphors, Red phosohor of YBO3:Eu3+ , like disclosed in, for examples, Xia Li et. al., Materials Research Bulletin, volume 39, issue 12, 4 October 2004, pages 1923 - 1930) ECS transaction, 33, 95 (2011), 2/ Jpn. J. Appl. Phys. 51 (2012) 062602, Phosphor Safari, 5 (2013), The 20th IDW, 797 (2013), J. Lumin., 129, 9 (2009) 1067, Tetsuhiko Isobe et.al., Development and Application of Nanophosphors, CMC Publishing Co., Ltd. 2012.11. can be used preferably.

- Quantum sized material

Preferably, the nanosized phosphor is a quantum sized material. As a quantum sized material, publically known any quantum sized material can be used in this way preferably. The type of quantum sized material is not particularly limited. The shape of quantum sized material is not particularly limited and includes sphere (dot), rod, disk, and other shapes.

According to the present invention, the term "quantum sized" means the size of the inorganic semiconductor material itself without ligands or another surface modification, which can show the quantum size effect.

Generally, quantum sized material such as quantum dot materials, and / or quantum rod materials can emit sharp vivid colored light due to quantum size effect.

In a preferred embodiment of the present invention, the quantum sized material can be selected from the group consisting of II-VI, III-V, IV-VI semiconductors and combinations of any of these.

More preferably, the quantum sized material can be selected from the groups consisting of Cds, CdSe, CdTe, ZnS, ZnSe, ZnTe, ZnO, GaAs, GaP, GaAs, GaSb, HgS, HgSe, HgSe, HgTe, InAs, InP, InSb, AlAs, AIP, AlSb, $Cu_2S$, $Cu_2Se$, CuInS2, $CuInSe_2$, $Cu_2(ZnSn)S_4$, $Cu_2(InGa)S_4$, $TiO_2$ alloys and a combination of any of these.

For example, for red emission use, CdSe rods / dots, CdSe dot in CdS rod, ZnSe dot in CdS rod, CdSe/ZnS rods / dots, InP rods / dots, CdSe/CdS rods / dots, ZnSe/CdS rods / dots, or a combination of any of these. For green emission use, such as CdSe rods, CdSe/ZnS rods, or combination of any of these, and for blue emission use, such as ZnSe, ZnS, ZnSe/ZnS core shell rods, or combination of any of these.

Examples of quantum rod material have been described in, for example, the international patent application laid-open No.WO2010/095140A. As a quantum dot, a publically available quantum dot, for example from Sigma-Aldrich, can be used preferably as desired.

Without wishing to be bound by theory, it is believed that light luminescence from dipole moment of the nanosized phosphor having elongated shape may lead to higher out-coupling efficiency than the out-coupling efficiency of spherical light emission from quantum dot, organic fluorescent material, and / or organic phosphorescent material, phosphor material.

In addition, it is believed that the long axis of the nanosized phosphor having elongated shape such as quantum rods can align in horizontal axis to a substrate surface on average with higher probability and their dipole moments also can align in horizontal axis to the substrate surface on average with higher probability.

According to the present invention, in case a quantum sized material needs to realize sharp vivid color(s) of the device with better out-coupling effect at the same time, a quantum rod material can be used more preferably.

The overall diameter of the said quantum sized material itself without any ligands or overcoat layer is in the range from 1 nm to 20 nm. More particularly, it is from 1 nm to 10 nm.

In some embodiments of the present invention, the quantum sized material such as a quantum rod and / or quantum dot material can further comprise a remaining surface ligand.

In some embodiments of the present invention, the outmost surface of the shell layer of the luminescent particle (100) can be over coated with one or more kinds of surface ligands.

Thus, one embodiment of the present invention, the nanosized phosphor further comprises a ligand covering the shell material.

Without wishing to be bound by theory it is believed that such surface ligands may lead to better dispersivity of the luminescent particle (100) in a solvent.

In a preferred embodiment of the present invention, the ligand can directly attached onto the shell material.

The surface ligands in common use include phosphines and phosphine oxides such as Trioctylphosphine oxide (TOPO), Trioctylphosphine (TOP), and Tributylphosphine (TBP); phosphonic acids such as Dodecylphosphonic acid (DDPA), Tridecylphosphonic acid (TDPA), Octadecylphosphonic acid (ODPA), and Hexylphosphonic acid (HPA); amines such as Dedecyl amine (DDA), Tetradecyl amine (TDA), Hexadecyl amine (HDA), and Octadecyl amine (ODA), thiols such as hexadecane thiol and hexane thiol; mercapto carboxylic acids such as mercapto propionic acid and mercaptoundecanoicacid; and a combination of any of these.

Examples of surface ligands have been described in, for example, the international patent application laid-open No. WO 2012/059931A.

In some embodiments of the present invention, the luminescent particle (100) can be the luminescent particle essentially consisting of a nanosized phosphor (110), a polymer material (120) covering the nanosized phosphor (110), and a shell layer (130) covering the polymer material (120), wherein the shell layer (130) is selected from the group consisting of a metal hydroxide, metal carbonate hydroxide, metal polyphosphate and a combination of any of these.

Or in some embodiments of the present invention, the luminescent particle (100) can be the luminescent particle essentially consisting of a nanosized phosphor (110), a polymer material (120) covering the nanosized phosphor (110), and a shell layer (130) covering the polymer material (120) and a ligand attached directory onto the shell layer (130), wherein the shell layer (130) is selected from the group consisting of a metal hydroxide, metal carbonate hydroxide, metal polyphosphate and a combination of any of these.

In one embodiment of the present invention, the luminescent particle (100) can be the luminescent particle consisting of a nanosized phosphor (110), a polymer material (120) covering the nanosized phosphor (110), and a shell layer (130) covering the polymer material (120), wherein the shell layer (130) is selected from the group consisting of a metal hydroxide, metal carbonate hydroxide, metal polyphosphate and a combination of any of these.

In one embodiment of the present invention, the luminescent particle (100) can be the luminescent particle consisting of a nanosized phosphor (110), a polymer material (120) covering the nanosized phosphor (110), and a shell layer (130) covering the polymer material (120) and a ligand attached directory onto the shell layer (130), wherein the shell layer (130) is selected from the group consisting of a metal hydroxide, metal carbonate hydroxide, metal polyphosphate and a combination of any of these.

In another aspect, the present invention further relates to use of the luminescent particle (100) in an optical device or biological imaging.

In one embodiment of the present invention, the optical device can be an optical film, for example, a color filter, color conversion film, remote phosphor tape, or another film or filter can be preferably used in an optical device.

In some embodiments of the present invention, the optical device can also be a liquid crystal display, Organic Light Emitting Diode (OLED), backlight unit for display, Light Emitting Diode (LED), Micro Electro Mechanical Systems (here in after "MEMS"), electro wetting display, or an electrophoretic display, lighting device, and / or a solar cell.

In the case of the optical device is a liquid crystal display, the luminescent particle (100) can be used in any type of liquid crystal display. For example, twisted nematic mode liquid crystal display, vertical alignment mode liquid crystal display, IPS mode liquid crystal display, guest host mode liquid crystal display.

- Ink formulation of the present invention

In another aspect, the present invention further relates to an ink formulation comprising the luminescent particle (100) and solvent.

In a preferred embodiment of the present invention, the solvent can be selected from the group consisting of purified water; ethylene glycol monoalkyl ethers, such as, ethylene glycol monomethyl ether, ethylene glycol monoethyl ether, ethylene glycol monopropyl ether, and ethylene glycol monobutyl ether; diethylene glycol dialkyl ethers, such as, diethylene glycol dimethyl ether, diethylene glycol diethyl ether, diethylene glycol dipropyl ether, and diethylene glycol dibutyl ether; ethylene glycol alkyl ether acetates, such as, methyl cellosolve acetate and ethyl cellosolve acetate; propylene glycol alkyl ether acetates, such as, propylene glycol monomethyl ether acetate (PGMEA), propylene glycol monoethyl ether acetate, and propylene glycol monopropyl ether acetate; aromatic hydrocarbons, such as, benzene, toluene and xylene; ketones, such as, methyl ethyl ketone, acetone, methyl amyl ketone, methyl isobutyl ketone, and cyclohexanone; alcohols, such as, ethanol, propanol, butanol, hexanol, cyclohexanol, ethylene glycol, and glycerin; esters, such as, ethyl 3-ethoxypropionate, methyl 3-methoxypropionate and ethyl lactate; and cyclic asters, such as, γ-butyrolactone; chlorinated hydrocarbons, such as chloroform, dichloromethane, chlorobenzene, dichlorobenzene. Those solvents are used singly or in combination of two or more, and the amount thereof depends on the coating method and the thickness of the coating.

More preferably, propylene glycol alkyl ether acetates, such as, propylene glycol monomethyl ether acetate (hereafter "PGMEA"), propylene glycol monoethyl ether acetate, or propylene glycol monopropyl ether acetate can be used. Even more preferably, PGMEA can be used.

The amount of the solvent in the photosensitive composition can be freely controlled according to the method of coating the composition. For example, if the composition is to be spray-coated, it can contain the solvent in an amount of 90 wt. % or more. Further, if a slit-coating method, which is often adopted in coating a large substrate, is to be carried out, the content of the solvent is normally 60 wt. % or more, preferably 70 wt. % or more.

- Scattering particles and / or reflective index adjusting materials In some embodiments of the present invention, the ink formulation can further comprise one or more of additives such as scattering particles, reflective index adjusting material, surfactant and a combination of any of these.

According to the present invention, as the light scattering particles, any type of publically known light scattering particles having different refractive index from the matrix material of the layer which includes the light scattering particles and can give Mie scattering effects, can be used preferably as desired.

For examples, small particles of inorganic oxides such as $SiO_2$, $SnO_2$, CuO, CoO, $Al_2O_3$ $TiO_2$, $Fe_2O_3$, $Y_2O_3$, ZnO, MgO; organic particles such as polymerized polystyrene, polymerized PMMA; inorganic hollow oxides such as hollow silica or a combination of any of these; can be used preferably.

Aforementioned light scattering particles can be used as the index adjusting material.

Preferably, the average particle diameter of the light scattering particles and or the reflective index adjusting material can be in the range from 350 nm to 5 $\mu$m.

Without wishing to be bound by theory, it is believed that more than 350 nm average particle diameter may lead to strong forward scattering caused by Mie scattering in a later, even if the refractive index difference between the light scattering particles and the layer matrix is as small as 0.1.

On the other hand, to obtain better layer forming properties by using the light scattering particles, maximum average particle diameter is 5 um or less, preferably. More preferably, from 500 nm to 2 $\mu$m.

- Coloring agent

In some embodiments of the present invention, the ink formulation can further comprise a coloring agent selected from the group consisting of a dye, pigment and a combination of any of these.

According to the present invention, as the coloring agent that can absorb a visible light can be selected from the group consisting of dye, pigment and a combination of any of these. Preferably, any type of publically known dye and / pigment for LCD color filter can be used in this way.

For examples, as shown in "Technologies on LCD Color Filter and Chemicals" CMC Publishing P. 53 (1998)" azo-chelate pigments, fused azo pigments, quinacridone pigments, isoindolinone pigments, perylene pigments perinone pigments, insoluble azo pigments, phthalocyanice pigments, dioxazine pigments, anthraquinone pigments, thioin pigments or a combination of any of these may be used.

- Surfactant

According to the present invention, any type of surfactant can be used preferably as desired.
The surfactants usable in the present invention are, for example, nonionic, anionic and amphoteric surfactants.

Examples of the nonionic surfactants include: polyoxyethylene alkyl ethers, such as, polyoxyethylene lauryl ether, polyoxyethylene oleyl ether and polyoxyethylene cetyl ether; polyoxyethylene fatty acid diethers; polyoxyethylene fatty acid monoethers; polyoxyethylene-polyoxypropylene block polymer; acetylene alcohol; acetylene glycol derivatives, such as, acetylene glycol, polyethoxyate of acetylene alcohol, and polyethoxyate of acetylene glycol; silicon-containing surfactants, such as, Fluorad ([trademark], manufactured by Sumitomo 3M Limited), MEGAFAC ([trademark], manufactured by DIC Corporation), and Surufuron ([trademark], manufactured by Asahi Glass Co., Ltd.); and organic siloxane surfactants, such as, KP341 ([trademark], manufactured by Shin-Etsu Chemical Co., Ltd.). Examples of the above acetylene glycols include: 3-methyl-1-butyne-3-ol, 3-methyl-1-pentyne-3-ol, 3,6-dimethyl-4-octyne-3,6-diol, 2,4,7,9-tetramethyl- 5-decyne-4,7-diol, 3,5-dimethyl-1-hexyne-3-ol, 2,5-dimethyl-3-hexyne-2,5-diol, and 2,5-dimethyl-2,5- hexanediol.
Examples of the anionic surfactants include: ammonium salts and organic amine salts of alkyldiphenylether disulfonic acids, ammonium salts and organic amine salts of alkyldiphenylether sulfonic acids, ammonium salts and organic amine salts of alkylbenzenesulfonic acids, ammonium salts and organic amine salts of polyoxyethylenealkylether sulfuric acids, and ammonium salts and organic amine salts of alkylsulfuric acids.

Further, examples of the amphoteric surfactants include 2-alkyl-N-carboxymethyl-N-hydroxyethyl imidazolium betaine, and laurylic acid amidopropyl hydroxy sulfone betaine.

- Polymer composition of the present invention

In another aspect, the present invention further relates to a polymer composition comprising the luminescent particle (100), and a polymer matrix.

Any publically known polymer matrix can be used preferably.
For example, one or more of polymers mentioned as the polymer material (120), and / or polysiloxanes like described in for example JP 2014-114176, WO 2012/157696 A1, WO 2013/151166 A can be used as the polymer matrix of the present invention preferably.

The amount of the luminescent particle (100) can be in the range from 0.01 to 99 weight parts based on total amount of the polymer composition, with preferably being in the range from 0.1 to 40 wt. % based on total amount of the polymer composition.

The amount of the polymer matrix can be in the range from 99.99 wt. % to 1 wt. % based on total amount of the polymer composition, with preferably being in the range from 99.9 wt. % to 60 wt. %.

Preferably, the polymer matrix can further contain one or more kinds of solvents, coloring agents, and / or scattering particles as described above in the ink formulation.

- Optical devices comprising the luminescent particle (100)

In another aspect, the invention relates to an optical device comprising the luminescent particle (100).

In some embodiments of the present invention, the optical device can be an optical film, for example, a color filter, color conversion film, remote phosphor tape, or another film or filter preferably can be used in an optical device.

In some embodiments of the present invention, the optical device can be a liquid crystal display, Organic Light Emitting Diode (OLED), backlight unit for display, Light Emitting Diode (LED), Micro Electro Mechanical Systems (here in after "MEMS"), electro wetting display, or an electrophoretic display, lighting device, and / or a solar cell.

In the case of the optical device is a liquid crystal display, the luminescent particle (100) can be used in any type of liquid crystal display. For example, twisted nematic mode liquid crystal display, vertical alignment mode liquid crystal display, IPS mode liquid crystal display, guest host mode liquid crystal display.

According to the present invention, the term "film" includes "layer" and "sheet" "tape" like structures.

In some embodiments of the present invention, the optical device can further comprise one or more of additives for example, scattering particles, reflective index adjusting material and a combination of any of these.

In some embodiments of the present invention, the optical device can further comprise coloring agent selected from the group consisting of a dye, pigment and a combination of any of these.

According to the present invention, one of the main requirements for the coloring agent is to absorb an excitation light from the light source which light excites the luminescent particle (100) in the optical device to improve color purity of light from the optical device. Therefore, commercially available yellow pigments for LCD color filter also can be used in a green sub color pixel if the optical film has the green sub color pixel.

In a preferred embodiment of the present invention, the optical film can be a homogeneous color conversion film like described in Fig. 3 or can comprise first and second sub color areas, like color filters, in which at least first sub color area emits light having longer peak wavelength than the second sub color areas when it is illuminated by light source.

In a preferred embodiment of the present invention, the optical film can comprise red sub color areas, green sub color areas and blue sub color areas.

In some embodiment of the present invention, the optical film can mainly consist of red sub color areas, green sub color areas and blue sub color areas like described in Fig. 2.

Even more preferably, in case of blue light emitting light source such as blue LED(s) is used, the blue sub color areas can be made without blue luminescent particle (100).

In some embodiments of the present invention, optionally, the optical film can further comprise a black matrix (hereafter "BM").

In a preferred embodiment, the BM can be placed in between the sub color areas like described in Fig. 2. In other words, sub color areas of the present invention can be marked out by one or more of the BM.

A material for the BM is not particularly limited. Well known materials, especially well known BM materials for color filters can be used preferably as desired. Such as black dye dispersed polymer composition, like described in JP 2008-260927A, WO 2013/031753A.

Fabrication method of the BM is not particularly limited, well known techniques can be used in this way. Such as, direct screen printing, photolithography, vapor deposition with mask.

In a preferred embodiment of the present invention, the optical device can embrace a light source.

According to the present invention, the type of light source in the optical device is not particularly limited. Preferably, UV or blue single color light source can be used.

More preferably, the light source emits light having peak wavelength in a blue light region, such as blue LED, CCFL, EL, or a combination of any of these, can be used.

In a preferred embodiment of the present invention, optionally, the optical device can embrace a light guiding plate to increase light uniformity from the light source.

In a preferred embodiment of the present invention, the optical device can further embrace a light scattering layer.

In some embodiments of the present invention, optionally, the optical device can further include a color filter layer. According to the present invention, as the color filter, any type of publically known color filter including red, green and blue sub color region for optical devices, such as LCD color filter, can be used in this way preferably.

In some embodiments of the present invention, optionally, the optical device can further comprise a selective light reflection layer.

According to the present invention, the term "light reflection" means reflecting at least around 60 % of incident light at a wavelength or a range of wavelength used during operation of a polarized light emissive device. Preferably, it is over 70 %, more preferably, over 75%, the most preferably, it is over 80 %.

A material for the selective light reflection layer is not particularly limited. Well known materials for a selective light reflection layer can be used preferably as desired.

According to the present invention, the selective light reflection layer can be single layer or multiple layers. In a preferred embodiment, the selective light reflection layer is selected from the group consisting of Al layer, Al + MgF$_2$ stacked layers, Al + SiO stacked layers, Al + dielectric multiple layer, Au layer, dielectric multiple layer, Cr + Au stacked layers; with the selective light reflection layer more preferably being Al layer, Al + MgF$_2$ stacked layers, Al + SiO stacked layers, cholesteric liquid crystal layer, stacked cholesteric liquid crystal layers.

Examples of cholesteric liquid crystal layers as a selective light reflection layer have been described in, for example, the international patent application laid-open No. WO 2013/156112A, WO 2011/107215 A.

In general, the methods of preparing the selective light reflection layer can vary as desired and selected from well-known techniques.

In some embodiments, the selective light reflection layer except for cholesteric liquid crystal layers can be prepared by a gas phase based coating process (such as Sputtering, Chemical Vapor Deposition, vapor deposition, flash evaporation), or a liquid-based coating process.

In case of the cholesteric liquid crystal layers, can be prepared by method described in, for example, WO 2013/156112A, or WO 2011/107215 A.

- Fabrication process

In another aspect, the present invention furthermore relates to method for preparing of the luminescent particle (100), wherein the method comprises the following sequential steps (a) to (b);

(a) mixing the nanosized phosphor and the polymer in solvent

(b) mixing the polymer covered nanosized phosphor obtained from step (a) and a precursor of the shell material in solvent

Preferably, the mixing condition in step (a) is at room temperature.

In a preferred embodiment of the present invention, the solvent in step (a) can be selected from the group consisting of purified water; ethylene glycol monoalkyl ethers, such as, ethylene glycol monomethyl ether, ethylene glycol monoethyl ether, ethylene glycol monopropyl ether, and ethylene glycol monobutyl ether; diethylene glycol dialkyl ethers, such as, diethylene glycol dimethyl ether, diethylene glycol diethyl ether, diethylene glycol dipropyl ether, and diethylene glycol dibutyl ether; ethylene glycol alkyl ether acetates, such as, methyl cellosolve acetate and ethyl cellosolve acetate; propylene glycol alkyl ether acetates, such as, propylene glycol monomethyl ether acetate (PGMEA), propylene glycol monoethyl ether acetate, and propylene glycol monopropyl ether acetate; aromatic hydrocarbons, such as, benzene, toluene and xylene; ketones, such as, methyl ethyl ketone, acetone, methyl amyl ketone, methyl isobutyl ketone, and cyclohexanone; alcohols, such as, ethanol, propanol, butanol, hexanol, cyclohexanol, ethylene glycol, and glycerin; esters, such as, ethyl 3-ethoxypropionate, methyl 3-methoxypropionate and ethyl lactate; and cyclic asters, such as, γ-butyrolactone; chlorinated hydrocarbons, such as chloroform, dichloromethane, chlorobenzene, dichlorobenzene and a combination of any of these. More preferably, chloroform, dichloromethane, tetrahydrofuran, ethanol, purified water or a combination of any of these.

In step (b), as a precursor of metal hydroxide, for example, tetraethyl orthosilicate (TEOS), methyl triethoxysilane (MTEOS), sodium silicate, lithium silicate, kalium silicate, aluminium isopropoxide, Tripropyl orthoaluminate Al (OC3H7)3 (TPOAI),Titanium alkoxide, vanadium alkoxide or a combination of any of these can be used preferably. As a precursor of Metal carbonate hydroxide, for example, Gd(NO)3·6H2O, Eu(NO)3·6H2O, Ce(NO)3·6H2O, or a combination of any of these; as a precursor of metal oxide polyphosphate, for example, Al(NO3)3·9H2O and polyphosphoric acid, Ca(NO3)2·4H2O and polyphosphoric acid or a combination of any of these can be used preferably.

In step (b), the type of solvent is not particularly limited.

One or more of hydrophilic solvents, or hydrophobic solvents together with one or more of surfactants can be used in this way preferably.

For example, purified water; ethylene glycol monoalkyl ethers, such as, ethylene glycol monomethyl ether, ethylene glycol monoethyl ether, ethylene glycol monopropyl ether, and ethylene glycol monobutyl ether; diethylene glycol dialkyl ethers, such as, diethylene glycol dimethyl ether, diethylene glycol diethyl ether, diethylene glycol dipropyl ether, and diethylene glycol dibutyl ether; ethylene glycol alkyl ether acetates, such as, methyl cellosolve acetate and ethyl cellosolve acetate; propylene glycol alkyl ether acetates, such as, propylene glycol monomethyl ether acetate (PGMEA), propylene glycol monoethyl ether acetate, and propylene glycol monopropyl ether acetate; aromatic hydrocarbons, such as, benzene, toluene and xylene; hexane, cycrohexane; ketones, such as, methyl ethyl ketone, acetone, methyl amyl ketone, methyl isobutyl ketone, and cyclohexanone; short chain alcohols, such as, ethanol, propanol, butanol, hexanol, cyclohexanol, ethylene glycol, and glycerin; esters, such as, ethyl 3-ethoxypropionate, methyl 3-methoxypropionate and ethyl lactate; and cyclic asters, such as, $\gamma$-butyrolactone; chlorinated hydrocarbons, such as chloroform, dichloromethane, chlorobenzene, dichlorobenzene and a combination of any of these.

More preferably, as a hydrophilic solvent, purified water, short chain alcohols, such as, ethanol, propanol, butanol, hexanol, cyclohexanol, ethylene glycol, and glycerin, or a combination of any of these can be used. And as a hydrophobic solvent, toluene, hexane, cycrohexane, or a combination of any of these can be used together with one or more of surfactants preferably.

As a surfactant, which can be used with hydrophilic solvent, any type of surfactant can be used preferably as desired.

For example, Igepal CO-520 (poly(oxyethylene)nonylphenyl ether), AOT (bis(2-ethylhexyl)sulfosuccinic acid sodium salt, Triton X-100 (octyl phonol ethoxylate) can be used together with one or more of hydrophilic solvents preferably.

In a preferred embodiment of the present invention, a catalyst also can be used in step (b) together with the precursor of the shell, to effectively fabricate the shell onto the polymer covered nanosized phosphor.

The type of the catalyst is not particularly limited. Publically known any alkaline, base or acid catalyst can be used preferably.

For example, NaOH, KOH, tetramethylammonium hydroxide, ammonia, hydrochloric acid, phosphoric acid, acetic acid, or a combination of any of these.

Fabrication process for a metal hydroxide, metal carbonate hydroxide, publically known techniques can be used preferably. For examples, like described in Ji-Guang Li, et. al., Chem. Mater. 2008, 20, 2274-2281, Weihua Di, et. al., Journal of Materials Chemistry, 2012, 22, 20641, and / or Yoshio Kobayashi, et. al., J Sol-Gel Sci Technol, 2010, 55; 79 - 85.

A fabrication process for a metal oxide polyphosphate shell, like described in for example, US 7,763,359 B2, Emilia Celma de Oliveira Lima et.al. Langmuir 1996, 12, 1701 - 1703, can be used preferably.

In another aspect, the present invention furthermore relates to method for preparing of the ink formulation, wherein the method comprises the step (x):

(x) mixing the luminescent particle and a solvent

In another aspect, the present invention furthermore relates to method for preparing of the polymer composition, wherein the method comprises the step (A):

(A) mixing the luminescent particle and a polymer

- Coating step

According to the present invention, to provide the luminescent particle (100), ink formulation, and / or the polymer composition onto a substrate to fabricate an optical device, any type of publically known coating method can be used preferably. For examples, ink jet printing, nozzle printing, immersion coating, gravure coating, roll coating, bar coating, brush coating, spray coating, doctor coating, flow coating, spin coating, and slit coating.

The invention is described in more detail in reference to the following examples, which are only illustrative and do not limit the scope of the invention.

Each feature disclosed in this specification, unless stated otherwise, may be replaced by alternative features serving the same, equivalent, or similar purpose. Thus, unless stated otherwise, each feature disclosed is but one example of a generic series of equivalent or similar features.

Definition of Terms

According to the present invention, the term "transparent" means at least around 60 % of incident light transmittal at the thickness used in a polarized light emissive device and at a wavelength or a range of wavelength used during operation of a polarized light emissive device. Preferably, it is over 70 %, more preferably, over 75%, the most preferably, it is over 80 %.

The term "fluorescent" is defined as the physical process of light emission by a substance that has absorbed light or other electromagnetic radiation. It is a form of luminescence. In most cases, the emitted light has a longer wavelength, and therefore lower energy, than the absorbed radiation.

The term "semiconductor" means a material which has electrical conductivity to a degree between that of a conductor (such as copper) and that of an insulator (such as glass) at room temperature.

The term "inorganic" means any material not containing carbon atoms or any compound that containing carbon atoms ionically bound to other atoms such as carbon monoxide, carbon dioxide, carbonates, cyanides, cyanates, carbides, and thiocyanates.

The term "emission" means the emission of electromagnetic waves by electron transitions in atoms and molecules.

The working examples 1 - 2 below provide descriptions of the present invention, as well as an in detail description of their fabrication.

**Working Examples**

**Working Example 1: Fabrication of luminescent particles having silicon oxide hydroxide shell layer**

- Production of polymer covered nanosized phosphor -

Solution of 750 mg polyethyleneimine polymer (Sigma-Aldrich) with 3 ml chloroform was added into solution of 100 mg of CdSe / CdS quantum sized material having elongated shape with 3 ml chloroform, and stirred overnight.

Then 16 ml cyclohexane was added into the obtained mixture solution to precipitate CdSe / CdS quantum sized material coated with polyethyleneimine out of the mixture solution. The obtained precipitate was separated by centrifuge and redispersed in 4 ml $CHCl_3$. After adding 10 ml of cyclohexane, precipitated CdSe / CdS quantum sized material coated with polyethyleneimine was separated by centrifuge, dried under vacuum condition and dispersed in 5 ml of purified water.

- Shell layer fabrication onto the polymer covered nanosized phosphor -

Purified water / ethanol solution (ratio of purified water: ethanol = 1: 4.43), and successively tetraethyl orthosilicate (TEOS) / ethanol solution were added into the obtained water dispersion with polymer covered quantum sized material.

Then, the silicon oxide hydroxide coating was initiated by rapidly injecting 0.1 mol / l NaOH aqueous solution into the quantum sized material / TEOS colloidal solution and settled for 24 hours.

Here, initial concentrations of polymer covered quantum sized material, water, NaOH and TEOS were 6.8 * $10^{-10}$, 8, 1 $10^{-4}$, 1 * $10^{-3}$ mol / l each respectively.

After the settlement, precipitation of the reaction mixture was centrifuged and dispersed in purified water. Finally, nanosized phosphor example 1 was obtained.

**Working Example 2: Fabrication of luminescent particles having metal carbonate hydroxide shell layer**

- Production of polymer covered nanosized phosphor -

Polymer covered nanosized phosphors were fabricated in the same manner as described in working example 1.

- Shell layer fabrication onto the polymer covered nanosized phosphor -

Urea (Kanto Chemical Co.), ethylenediaminetetraacetic acid (EDTA, Sigma-Aldrich) aqueous solution, and gadolinium nitrate hexahydrate aqueous solution (Kanto Chemical Co.) were successively added into purified water, and settled for three hours at 80 °C.

Initial concentrations of purified water; polymer covered quantum sized material, urea, EDTA and Gadolinium nitrate were 55.5, 6.8 * $10^{-10}$, 5 * $10^{-1}$, 1 * $10^{-4}$, 1 * $10^{-3}$ mol / l, each respectively.

Then, the polymer covered quantum sized material with $Gd(OH) CO_3$ shell was isolated from the reaction mixture by centrifuge, and dispersed in water for TEM and PLQY analysis. Then, nanosized phosphor example 2 was obtained.

**Comparative example: Fabrication of luminescent particles**

- Production of D-penicillamine (DPA) covered nanosized phosphor -

Solution of 32 mg of CdSe / CdS quantum sized material having elongated shape with 2 ml chloroform and

100 μl of 25 wt. % of tetramethylammonium hydroxide solution in methanol were added into solution of 93 mg D-penicillamine (DPA) (Sigma-Aldrich) with 2 ml of purified water, and stirred at 1,000 rpm for three hours at room temperature.

In the aforementioned procedure, the quantum sized material was extracted from chloroform phase to water phase by successful ligand exchange of the quantum sized material with DPA.

Then the chloroform was evaporated.

After the evaporation of chloroform, DPA covered quantum sized material in purified water was ultracentrifuged at 24,000 rpm for 1 hour and isolated from water.

The isolated DPA covered quantum sized material was re-dispersed in 1.42 ml of purified water.

- Shell layer fabrication onto D-penicillamine (DPA) covered nanosized phosphor -

Shell layer fabrication was carried out in the same manner as described in working example 1, except for D-penicillamine (DPA) covered nanosized phosphor was used instead of polymer covered nanosized phosphor.

Then, comparative example was obtained.

**Working Example 3: Transmission Electron Microscope (TEM) analysis and Photoluminescence Quantum Yield (PLQY) measurement**

TEM analysis

- Sample 1

Aqueous dispersion of the polymer covered quantum sized material with shell layer obtained from working example 1 was casted onto a cupper mesh and dried for 2 hours in an ambient air condition at room temperature then further dried for 3 hours in vacuum.

- Sample 2

Aqueous dispersion of polymer covered quantum sized material with metal carbonate hydroxide shell layer obtained from working example 2 was also casted onto a cupper mesh and dried for 2 hours in an ambient air condition at room temperature then further dried for 3 hours in vacuum.

- Sample 3

Sample 3 was prepared in the same manner as described in above sample 1 in working example 3 except for D-penicillamine (DPA) covered nanosized phosphor obtained in comparative example was used instead of the polymer covered quantum sized material with shall layer from working example 1.

Then, TEM images of sample 1, 2 and 3 were measured with JEOL JEM-2000FX.

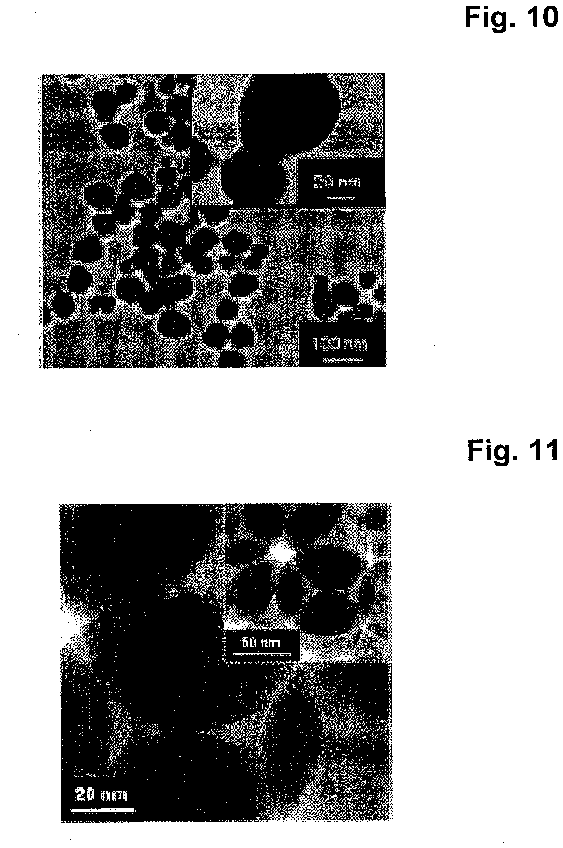

Figs. 9 to 11 show TEM images of samples 1 to 3 each respectively.

Photoluminescence quantum yield (PLQY) measurement

Photoluminescence quantum yield (PLQY) of polymer covered quantum sized materials with shall layer in aqueous solution obtained from working example 1, 2 and comparative example were measured with the absolute photoluminescence quantum yields measurement system C9920 - 02G (Hamamatsu photonics Co.).

According to the present invention, the quantum yield was evaluated with the following formula:

$$\text{Quantum yield} = \text{emitted photon number from the sample} / \text{absorbed photon number in the sample}.$$

And the relative PLQY was calculated with the following formula:

Relative PLQY = evaluated PLQY of working example 1, 2 or comparative example at 365 nm or 470 nm absorption wavelength / PLQY of working example 1 at 365 nm of absorption wavelength.

Table 1 shows PLQY of polymer covered quantum sized material with shell layer.

**Table 1**

| Absorption light wavelength (nm) | Working example 1 (relative PLQY) | Working example 2 (relative PLQY) | Comparative example (relative PLQY) |
|---|---|---|---|
| 365 | 1 | 1 | 0.39 |
| 470 | 1 | 1 | 0.39 |

**Claims**

1.  A luminescent particle (100), wherein the luminescent particle (100) comprises a nanosized phosphor (110), a polymer material (120) covering the nanosized phosphor (110), and a shell layer (130) covering the polymer material (120), wherein the shell layer (130) is selected from the group consisting of a metal hydroxide, metal carbonate hydroxide, metal polyphosphate and a combination of any of these.

2.  The luminescent particle (100) according to claim 1, wherein the weight average molecular weight (Mw) of the polymer (120) is in the range from 1000 to 100000.

3.  The luminescent particle (100) according to claim 1 or 2, wherein the polymer (120) further comprises an anchoring group selected from the group consisting of phosphate, phosphine, phosphine oxide, phosphonate, thiol, amino, carboxylate, carboxylic ester, hetero cycle, silane, sulfonate, hydroxyl and a combination of any of these.

4.  The luminescent particle (100) according to any one or more of claims 1 to 3, wherein the anchoring group is amino, phosphate, carboxylate, or a combination of any of these.

5.  The luminescent particle (100) according to any one or more of claims 1 to 4, wherein the polymer (120) is a branched polymer.

6.  The luminescent particle (100) according to any one or more of claims 1 to 5, wherein the nanosized phosphor (110) is a nanosized inorganic phosphor, or a quantum sized material.

7.  The luminescent particle (100) according to any one or more of claims 1 to 6, wherein the nanosized phosphor (110) further comprises a ligand (140) covering the shell layer (130).

8.  Use of the luminescent particle (100) according to any one or more of claims 1 to 7, in an optical device or biological imaging.

9.  Ink formulation comprising the luminescent particle (100) according to any one or more of claims 1 to 7, and solvent.

10. A polymer composition comprising the luminescent particle (100) according to any one or more of claims 1 to 7, and a polymer matrix.

11. An optical device comprising the luminescent particle (100) according to any one or more of claims 1 to 7.

12. Method for preparing of the luminescent particle (100) according to any one or more of claims 1 to 7, wherein the method comprises the following sequential steps (a) and (b);

    (a) mixing the nanosized phosphor and the polymer in solvent
    (b) mixing the polymer covered nanosized phosphor obtained from step (a) and a precursor of the shell layer in solvent.

**13.** Method for preparing the ink formulation according to claim 9, wherein the method comprises the step (x):
(x) mixing the luminescent particle and a solvent.

**14.** Method for preparing the polymer composition according to claim 10, wherein the method comprises the step (A):

(A) mixing the luminescent particle and a polymer.

**Patentansprüche**

**1.** Lumineszierendes Teilchen (100), wobei das lumineszierende Teilchen (100) einen nanoskaligen Leuchtstoff (110), ein den nanoskaligen Leuchtstoff (110) bedeckendes Polymermaterial (120) und eine das Polymermaterial (120) bedeckende Hüllschicht (130) enthält, wobei die Hüllschicht (130) aus der Gruppe bestehend aus einem Metallhydroxid, Metallcarbonat-hydroxid, Metallpolyphosphat und einer beliebigen Kombination hiervon ausgewählt ist.

**2.** Lumineszierendes Teilchen (100) nach Anspruch 1, wobei das gewichtsmittlere Molekulargewicht (Mw) des Polymers (120) im Bereich von 1000 bis 100000 liegt.

**3.** Lumineszierendes Teilchen (100) nach Anspruch 1 oder 2, wobei das Polymer (120) ferner eine Ankergruppe enthält, die aus der Gruppe bestehend aus Phosphat, Phosphin, Phosphinoxid, Phosphonat, Thiol, Amino, Carboxylat, Carbonsäureester, Heterocyclus, Silan, Sulfonat, Hydroxyl und einer beliebigen Kombination hiervon ausgewählt ist.

**4.** Lumineszierendes Teilchen (100) nach einem oder mehreren der Ansprüche 1 bis 3, wobei es sich bei der Ankergruppe um Amino, Phosphat, Carboxylat oder eine beliebige Kombination hiervon handelt.

**5.** Lumineszierendes Teilchen (100) nach einem oder mehreren der Ansprüche 1 bis 4, wobei das Polymer (120) ein verzweigtes Polymer ist.

**6.** Lumineszierendes Teilchen (100) nach einem oder mehreren der Ansprüche 1 bis 5, wobei es sich bei dem nanoskaligen Leuchtstoff (110) um einen nanoskaligen anorganischen Leuchtstoff oder ein Material im Quantenmaßstab handelt.

**7.** Lumineszierendes Teilchen (100) nach einem oder mehreren der Ansprüche 1 bis 6, wobei der nanoskalige Leuchtstoff (110) ferner einen die Hüllschicht (130) bedeckenden Liganden (140) enthält.

**8.** Verwendung des lumineszierenden Teilchens (100) nach einem oder mehreren der Ansprüche 1 bis 7 in einer optischen Vorrichtung oder biologischen Bildgebung.

**9.** Druckfarbenformulierung enthaltend das lumineszierende Teilchen (100) nach einem oder mehreren der Ansprüche 1 bis 7 und Lösungsmittel.

**10.** Polymerzusammensetzung enthaltend das lumineszierende Teilchen (100) nach einem oder mehreren der Ansprüche 1 bis 7 und eine Polymermatrix.

**11.** Optische Vorrichtung enthaltend das lumineszierende Teilchen (100) nach einem oder mehreren der Ansprüche 1 bis 7.

**12.** Verfahren zur Herstellung des lumineszierenden Teilchens (100) nach einem oder mehreren der Ansprüche 1 bis 7, wobei das Verfahren die folgenden aufeinanderfolgenden Schritte (a) und (b) umfasst:

(a) Mischen des nanoskaligen Leuchtstoffs und des Polymers in Lösungsmittel
(b) Mischen des aus Schritt (a) erhaltenen polymerbedeckten nanoskaligen Leuchtstoffs und eines Vorläufers der Hüllschicht in Lösungsmittel.

**13.** Verfahren zur Herstellung der Druckfarbenformulierung nach Anspruch 9, wobei das Verfahren den Schritt (x) umfasst:

(x) Mischen des lumineszierenden Teilchens und eines Lösungsmittels.

**14.** Verfahren zur Herstellung der Polymerzusammensetzung nach Anspruch 10, wobei das Verfahren den Schritt (A) umfasst:

(A) Mischen des lumineszierenden Teilchens und eines Polymers.

**Revendications**

**1.** Particule luminescente (100), dans laquelle la particule luminescente (100) comprend un phosphore nanométrique (110), un matériau de polymère (120) qui recouvre le phosphore nanométrique (110), et une couche d'enveloppe (130) qui recouvre le matériau de polymère (120), dans laquelle la couche d'enveloppe (130) est sélectionnée parmi le groupe qui est constitué par un hydroxyde métallique, un hydroxyde de carbonate métallique, un polyphosphate métallique et une combinaison de quelconques de ceux-ci.

**2.** Particule luminescente (100) selon la revendication 1, dans laquelle le poids moléculaire moyen en poids (Mw) du matériau de polymère (120) est dans la plage qui va de 1 000 à 100 000.

**3.** Particule luminescente (100) selon la revendication 1 ou 2, dans laquelle le matériau de polymère (120) comprend en outre un groupe d'ancrage qui est sélectionné parmi le groupe qui est constitué par le phosphate, la phosphine, l'oxyde de phosphine, le phosphonate, le thiol, l'amino, le carboxylate, l'ester carboxylique, l'hétérocycle, le silane, le sulfonate, l'hydroxyle et une combinaison de quelconques de ceux-ci.

**4.** Particule luminescente (100) selon l'une quelconque ou plusieurs quelconques des revendications 1 à 3, dans laquelle le groupe d'ancrages est l'amino, le phosphate, le carboxylate, ou une combinaison de quelconques de ceux-ci.

**5.** Particule luminescente (100) selon l'une quelconque ou plusieurs quelconques des revendications 1 à 4, dans laquelle le matériau de polymère (120) est un polymère ramifié.

**6.** Particule luminescente (100) selon l'une quelconque ou plusieurs quelconques des revendications 1 à 5, dans laquelle le phosphore nanométrique (110) est un phosphore inorganique nanométrique ou un matériau de taille quantique.

**7.** Particule luminescente (100) selon l'une quelconque ou plusieurs quelconques des revendications 1 à 6, dans laquelle le phosphore nanométrique (110) comprend en outre un ligand (140) qui recouvre la couche d'enveloppe (130).

**8.** Utilisation de la particule luminescente (100) selon l'une quelconque ou plusieurs quelconques des revendications 1 à 7, dans un dispositif optique ou lors d'une imagerie biologique.

**9.** Formulation d'encre comprenant la particule luminescente (100) selon l'une quelconque ou plusieurs quelconques des revendications 1 à 7, et un solvant.

**10.** Composition de polymère comprenant la particule luminescente (100) selon l'une quelconque ou plusieurs quelconques des revendications 1 à 7, et une matrice polymère.

**11.** Dispositif optique comprenant la particule luminescente (100) selon l'une quelconque ou plusieurs quelconques des revendications 1 à 7.

**12.** Procédé pour préparer la particule luminescente (100) selon l'une quelconque ou plusieurs quelconques des revendications 1 à 7, dans lequel le procédé comprend les étapes séquentielles qui suivent (a) et (b) :

(a) le mélange du phosphore nanométrique et du polymère dans un solvant ; et
(b) le mélange du phosphore nanométrique recouvert de polymère qui est obtenu au niveau de l'étape (a) et d'un précurseur de la couche d'enveloppe dans un solvant.

**13.** Procédé pour préparer la formulation d'encre selon la revendication 9, dans lequel le procédé comprend l'étape (x) :
(x) le mélange de la particule luminescente et d'un solvant.

**14.** Procédé pour préparer la composition de polymère selon la revendication 10, dans lequel le procédé comprend l'étape (A) :

(A) le mélange de la particule luminescente et d'un polymère.

**Fig. 1**

**Fig. 2**

## Fig. 3

## Fig. 4

**Fig. 5**

# Fig. 6

600

621
624
640
620
622
623
622
621

R G B

611 613 614 611

610

614 613 612

630

**Fig. 7**

700

**Fig. 8**

**Fig. 9**

**Fig. 10**

**Fig. 11**

## REFERENCES CITED IN THE DESCRIPTION

**Patent documents cited in the description**

- JP 4568862 B
- WO 2013035362 A1
- WO 2007034877 A1
- WO 2014140936 A2
- WO 2011036447 A1
- CN 103911142 A
- WO 2010095140 A
- WO 2012059931 A
- JP 2014114176 A
- WO 2012157696 A1
- WO 2013151166 A
- JP 2008260927 A
- WO 2013031753 A
- WO 2013156112 A
- WO 2011107215 A
- US 7763359 B2

**Non-patent literature cited in the description**

- **FRANCESCA PIETRA et al.** *Chem. Mater.,* 2013, vol. 25, 3427-3434
- **YOSHIO KOBAYASHI et al.** *J Sol-Gel Sci Technol,* 2010, vol. 55, 79-85
- **YOSHIO KOBAYASHI et al.** *J Sol-Gel Sci Technol,* 2013, vol. 66, 31-37
- **MITSURU UEDA.** *Landfall,* 2013, vol. 77, 16-21
- **XIA LI.** *Materials Research Bulletin,* 04 October 2004, vol. 39 (12), 1923-1930
- *ECS transaction,* 2011, vol. 33 (95), 2
- *Jpn. J. Appl. Phys.,* 2012, vol. 51, 062602
- *Phosphor Safari,* 2013, vol. 5
- *The 20th IDW,* 2013, vol. 797
- *J. Lumin.,* 2009, vol. 129 (9), 1067
- **TETSUHIKO ISOBE.** Development and Application of Nanophosphors. CMC Publishing Co., Ltd, November 2012
- Technologies on LCD Color Filter and Chemicals. CMC Publishing, 1998, 53
- **JI-GUANG LI.** *Chem. Mater.,* 2008, vol. 20, 2274-2281
- **WEIHUA DI.** *Journal of Materials Chemistry,* 2012, vol. 22, 20641
- **YOSHIO KOBAYASHI.** *J Sol-Gel Sci Technol,* 2010, vol. 55, 79-85
- **EMILIA CELMA DE OLIVEIRA LIMA.** *Langmuir,* 1996, vol. 12, 1701-1703